# EUROPEAN PATENT APPLICATION

(11) **EP 1 676 600 A1**
(43) Date of publication of application: **05.07.2006**
(21) Application number: 05026645.1
(22) Date of filing: 06.12.2005
(51) Int. Cl.: A61N 1/32, A61H 39/00, A61H 7/00

(54) **Device facilitating easy operation of massage**

(30) Priority: 28.12.2004 CN 200420120536
(71) Applicant: Tsai, Wei-Ting, Chiayi City (TW)
(72) Inventor: Tsai, Wei-Ting, Chiayi City (TW)
(74) Representative: Volpert, Marcus

(57) **Abstract**

A device for health and beauty care comprises an energy wave generator (1) consisting of a microprocessor (101), a signal generator (102), a filter (103), an attenuator (104), a gain amplifier (106), an exciter (107), a loop detector (108) and a voltage display (14). The surface of the energy wave generator (1) is provided with a plurality of sockets (10) and knobs (11,12). The sockets (10) are for connecting a conducting wire (13) with a free end separated into a positive-polarity connector (130) and a negative-polarity connector (131). The device for health and beauty care further comprises two connecting plates (2,3) respectively connected to the positive-polarity and the negative-polarity connectors (130,131) of the conducting wire (13). Each of the connecting plates (2,3) further includes an insulting pad (4). To use the present invention, a person places his or her bare feet of on the connecting plate (2), and the massagist stands on the connecting plate (3) barefooted, thereby forming a conducting medium and enhancing the massage effect by the electric energy wave.

## Description

### Field of the Invention :

The present invention relates to devices for health care, more particularly to a device for not only health but also beauty care.

### Background of the Invention :

The devices for health care of the prior art produces a curing effect by a low electric current similar to electric-potential stimulating acupuncture cure for not only easing up chronic ailments and pains but also providing a beauty care. However, the conventional electro-stimulating acupuncture cure is applied to fixed acupuncture points, and re-installment of the electrode plates is needed when acupuncture points being massaged are changed. Therefore, massage over as large body area is impossible. Further, conventional massage body care is applied to a person lying on a massage bed, while the massagist stands alongside (near the person's head or feet). The massagist will apply massage to various portions of the person. To achieve a lasting and therefore an effective massage effect, this posture is easy to cause body fatigue to the massagist and may bring hazards to his or her joints. Accordingly, electro-stimulating massage devices are invented to enhance the massage strength to the person, while the massagist has to touch the person's body gently. However, the conventional devices may apply to the person a high voltage, which may frighten or disturb the person.

### Summary of the Invention :

The primary objective of the present invention is to provide a device for health and beauty care functioning at low and safe electric current and facilitating easy operation of massage for beauty care.

A device for health and beauty care of the present invention is easy to operate, whereby a massagist can achieve an effective massage without exerting much force on a person being taken care of.

The various objects and advantages of the present invention will be more readily understood from the following detailed description when read in conjunction with the appended drawings.

### Brief Description of the Drawings :

Fig. 1 is an exploded perspective view of a device for health and beauty care of the present invention.
Fig. 2 is a block diagram of the energy wave generator of the device for health and beauty care in Fig. 1.
Fig. 3 illustrates a first application of a device for health and beauty care of the present invention.
Fig. 4 illustrates a second application of a device for health and beauty care of the present invention.
Fig. 5 illustrates a third application of a device for health and beauty care of the present invention.

### Detailed Description of the Preferred Embodiments:

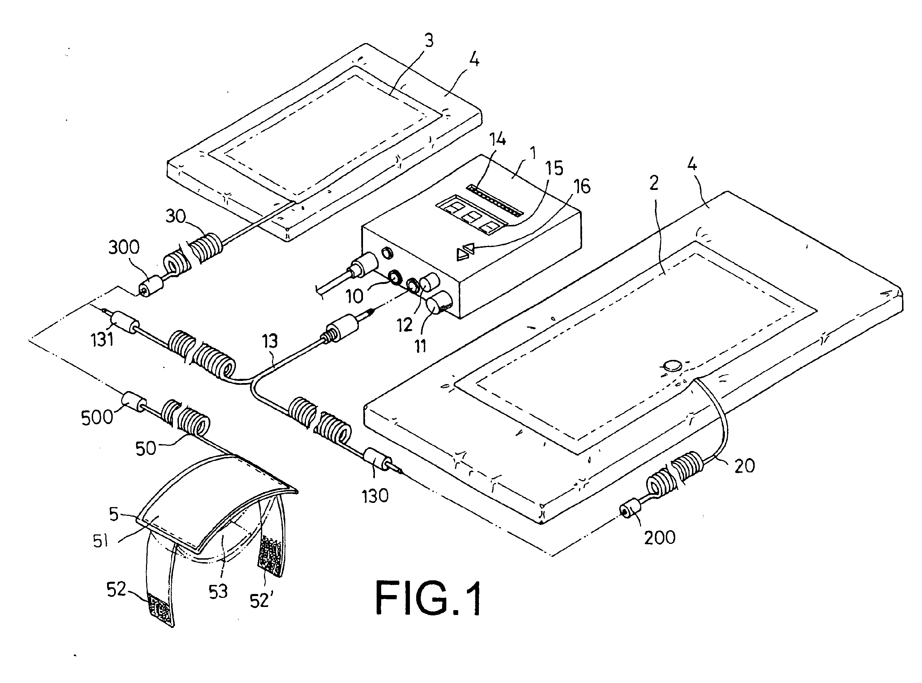

Referring to Figs. 1 to 5, a device for health and beauty care of the present invention comprises an energy wave generator 1, two conducting plates 2,3 and a conducting sleeve 5. The energy wave generator 1 further comprises a plurality of sockets 10, a switch knob 11 and an adjustment knob 12. The sockets 10 are for the insertion of a conducting wire 13. The free terminal of the conducting wire 13 is provided with a positive-polarity connector 130 and negative-polarity connector 131. The switch knob 11 controls the power supply and the associated current, whereas the adjustment knob 12 adjusts the amplitude and frequency of the massage wave. The energy wave generator 1 further includes a voltage display 14 indicating the magnitude of the voltage, a function display 15 and an input selection key 16.

The two conducting plates 2,3 are connected to the energy wave generator 1 through wires 20, 30, which is respectively provided with connectors 200, 300. The connectors 200, 300 are respectively connected to the positive-polarity connector 130 and the negative-polarity connector 131 of the conducting wire 13 extended from the energy wave generator 1. Further, the bottom sides of the two conducting plates 2,3 are respectively provided with insulating pads 4.

The conducting sleeve 5 is connected to the energy wave generator 1 through the conducting wire 13. A wire 50 is extended from the conducting sleeve 5, having a connector 500 installed at the free end thereof for the connection between the wire 50 and either of the positive-polarity connector 130 and negative-polarity connector 131 of the conducting wire 13. The inner wall of the conducting sleeve 5 is provided with a conducting plate 51, whereas the outer wall of the conducting sleeve 5 is provided with is provided with a pair of fastener straps 52, 52', whereby the fastener straps 52, 52' will form a glove portion 53 after their connection. The conducting sleeve 5 is attached to a hand for a healthcare massage. The interior of the conducting sleeve 5 is made of insulating materials.

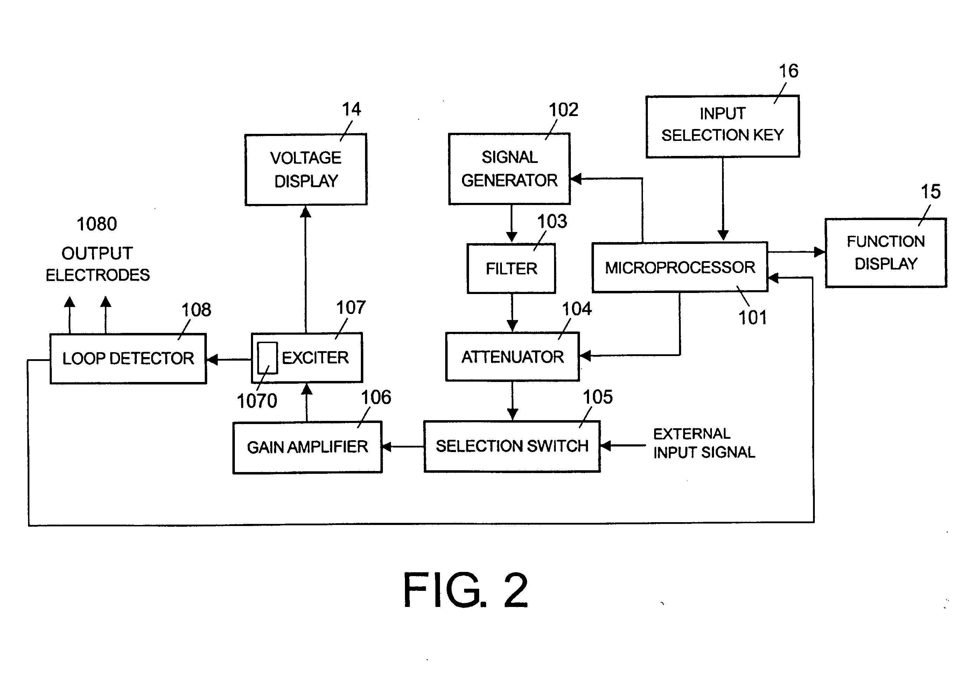

Refer to Fig.2 for a block diagram for the energy wave generator 1 of the device for health and beauty care. The energy wave generator 1 further comprises a microprocessor 101 storing a plurality of data sets, a signal generator 102 receiving the data sets from the microprocessor 101 and transforming the digital sets to analogy signals, a filter 103 removing sharp noises in the analogy signals generated in the signal generator 102, an attenuator 104 controlled by the microprocessor 101 to adjust the frequency of the filtered signals, a gain amplifier 106 for adjusting the magnitude of an applied voltage, an exciter 107 connected to an output terminal of the gain amplifier 106 for amplifying signals from the gain amplifier 106, a loop detector 108 connected to the exciter 107 for converting one of the analogy signals to a digital signal and feeding back a signal to the microprocessor 101, and a voltage display 14 connected to the exciter 107 for displaying voltage of an output electric signal. The input port of the attenuator 104 is further provided with a selection switch 105. The gain amplifier 106 is connected to either the attenuator 104 or the signal selection switch 105 that is connected to the attenuator 104. The output terminal further includes a limiter 1070 for restricting an output current within a predetermined range. The limiter 1070 is a device of positive temperature coefficient that produces larger reactance as the current increased, whereby the current falls into a safe range for human tolerance. An output terminal of the exciter 107 is equipped with a set of electrodes 1080 connected to sockets 10 and controlled by the microprocessor 101.

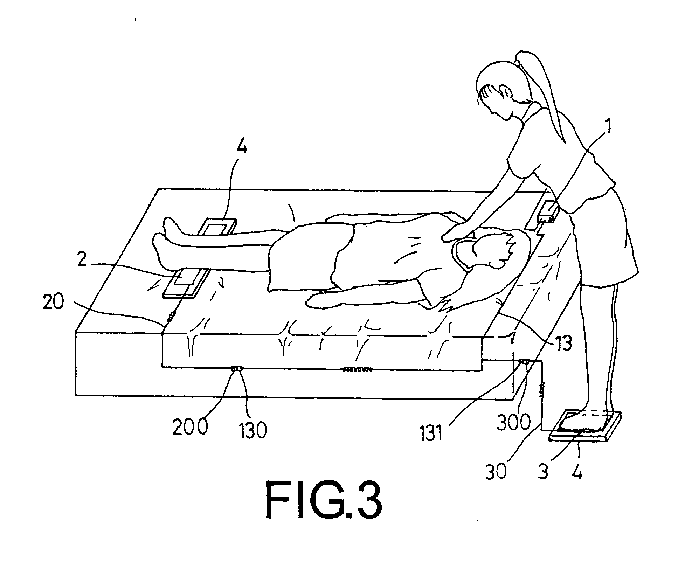

As shown in Figs. 1 and 3, the operation of the device for health and beauty care begins with the connection between the insertion terminal 200 of the wire 20 from the conducting plate 2 and the positive-polarity connector 130 of the conducting wire 13 from the energy wave generator 1. Then, the connector 300 of the wire 30 from the conducting plate 3 and the negative-polarity connector 131 of the conducting wire 13 from the energy wave generator 1 are connected. The conducting plates 2,3 are mounted on respective insulating pads 4, so that the feet of a person to be massaged and the massagist can be respectively placed on the conducting plates 2,3. The energy wave generator 1 is then charged, and the microprocessor 101 therein will determine if an electric signal will be sent. If the answer is negative, the microprocessor 101 will set a built-in data set of voltage, current, waveform and frequency. If the answer is positive, the energy wave generator 1 will determine if the output electrodes form a loop by detecting the load across the output electrodes. If a loop does not form, the potential difference across the output electrodes will be kept low. If a loop is formed, the potential difference across the output electrodes will be increased gradually to a predetermined voltage, in accordance with a built-in data set of voltage, current, waveform and frequency. Thereby, a user will be frightened by an initial touch with the output electrodes. At the same time, the massagist can adjust the amplitude and frequency of the signal. Basically, the massagist plays the role of a conducting medium between the energy wave generator 1 and the person being massaged when the massagist touches the person by hands.

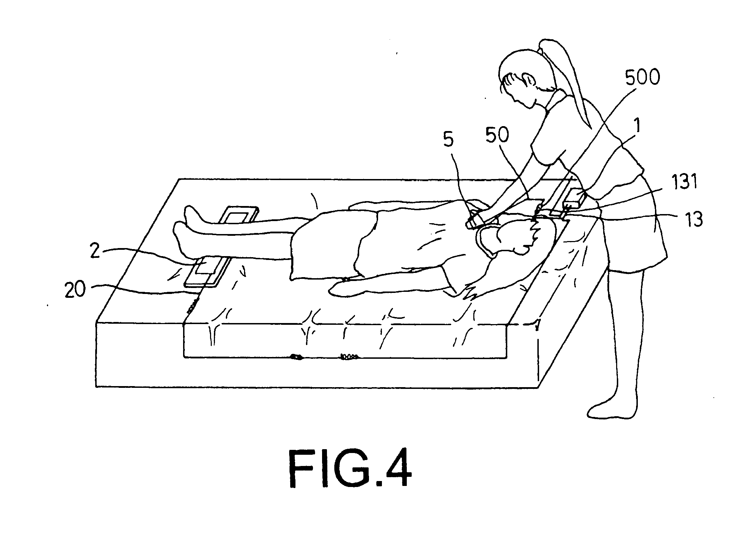

Referring to Fig. 4, a conducting sleeve 5 disposed on one hand of a massagist can replace the conducting plate 3 where the massagist stands on barefooted. In this case, the massagist is not the conducting medium. The connector 500 of the wire 50 from the conducting sleeve 5 is connected to the negative-polarity connector 131 of the wire 13 from the energy wave generator 1. The fastener straps 52, 52' on the back side of the conducting sleeve 5 are then connected so as to encircle the hand of the massagist, whereby the conducting plate 51 on the conducting sleeve 5 can be applied to a person, forming an effect similar to massage.

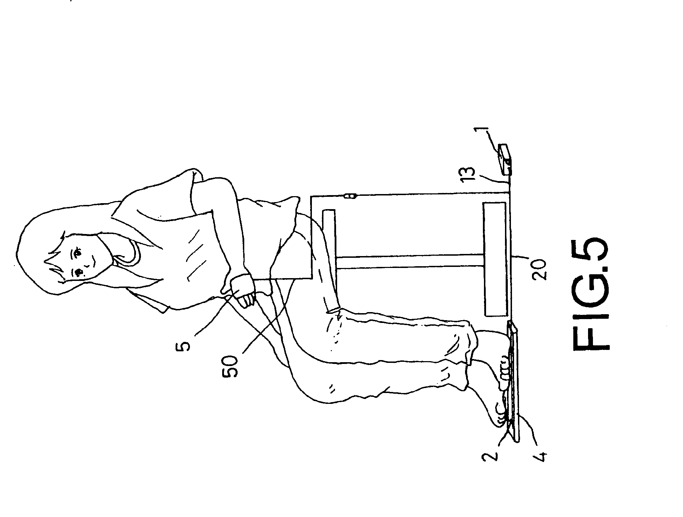

Referring to Fig. 5, a person can operate the device alone, by stepping on the conducting plate 2 barefooted and inserting his or her hand into the conducting sleeve 5. The person then uses the conducting sleeve 5 to touch any body portion to achieve a massage effect.

Further, the input selection key 16 and the function display 15 connected to the microprocessor 101 can be used by a person to modify the built-in data sets therein.

Therefore, a device for health and beauty care of the present invention has the following advantages.
1. The present invention utilizes electro-stimulating massage that can reduce the body fatigue of a massagist or a person performing beauty care.
2. The present invention is easy to operate and is therefore practical.
3. The present invention uses low and safe electric current, which avoids the point-wise heat and pain of the conventional electro-stimulating devices.
4. The present invention is capable of detecting loop formation and gradually increasing the amplitude of a wave signal, thereby prevent the shock felt by a user when firstly touches the device.
5. The present invention is flexible that the amplitude and frequency of the wave signal can be freely adjusted during an operation, by the switch knob and the adjustment knob.
6. The massage provided by the present invention can stimulate body cells for improving body immunity, blood vessel contraction and metabolism, achieving an effect of not only health care but also beauty care.
7. The present invention can increase the electric potential of body cell membranes and lessen the imbalance between the ionic concentrations outside and inside a cell. Thereby, the free radicals in a body can be removed, and the blood circulation can be enhanced.
8. Since the present invention can improve body metabolism, the body fat and the associated hazardous materials can be removed more easily, achieving an effect of beauty care.

The present invention is thus described, and it will be obvious that the same may be varied in many ways. Such variations are not to be regarded as a departure from the spirit and scope of the present invention, and all such modifications as would be obvious to one skilled in the art are intended to be included within the scope of the following claims.

## Claims

1. A device for health and beauty care, comprising two conducting plates and an energy wave generator, said an energy wave generator further comprising:
a microprocessor storing a plurality of data sets;
a signal generator receiving said data sets of said microprocessor and transforming said digital sets to analogy signals;
a filter removing sharp noises in said analogy signals generated in said signal generator;
an attenuator controlled by said microprocessor to adjust the frequency of said filtered signals;
a gain amplifier for adjusting the magnitude of an applied voltage;
an exciter connected to an output terminal of said gain amplifier for amplifying signals from said gain amplifier, said output terminal further including a limiter for restricting an output current within a predetermined range;
a loop detector connected to said exciter for converting one of said analogy signals to a digital signal and feeding back a signal to said microprocessor, said a loop detector having an output terminal equipped with a set of electrodes respectively connected to said two conducting plates; and
a voltage display connected to said exciter for displaying voltage of an output electric signal.

2. The device for health and beauty care of claim 1 wherein said energy wave generator has a signal selection switch between said attenuator and said gain amplifier for selecting an external input signal.

3. The device for health and beauty care of claim 1 wherein said limiter connected to said output terminal of said exciter is a device of positive temperature coefficient.

4. The device for health and beauty care of claim 1 wherein said microprocessor of said energy wave generator has an input selection key and a function display for selecting predetermined stored signal data therein.

5. A device for health and beauty care, comprising:
an energy wave generator provided with a plurality of sockets, switch knobs and adjustment knobs, said sockets being for connecting a conducting wire, a free end of said conducting wire being provided with a positive-polarity connector and a negative-polarity connector, said switch knobs being for switching power and adjusting electric current, said adjustment knobs adjusting the amplitude and frequency of the massage wave; and
two conducting plates connected to said energy wave generator through wires, said wires being connected to said conducting wire by insertion terminals thereon, each of said two conducting plates being provided with an insulating pad at a bottom side thereon.

6. The device for health and beauty care of claim 5 wherein one of said two conducting plates is replaced by a conducting sleeve having a wire connected to said conducting wire of said energy wave generator; said conducting sleeve being provided with a conducting plate on an outer surface thereon; an inner space of said conducting sleeve forming a glove portion.
